# EUROPEAN PATENT APPLICATION

(11) **EP 3 081 209 A1**
(43) Date of publication of application: **19.10.2016**
(21) Application number: 15001048.6
(22) Date of filing: 13.04.2015
(51) Int. Cl.: A61K 8/73, A61Q 19/08

(54) **SKIN CARE COMPOSITIONS COMPRISING MICROFIBRILLATED CELLULOSE**

(71) Applicant: Borregaard AS, 1721 Sarpsborg (NO)
(72) Inventor: Blell, Rebecca, 1701 Sarpsborg (NO); Nygård Vold, Inger Mari, 1701 Sarpsborg (NO); Øvrebrø, Hans Henrik, 1701 Sarpsborg (NO); Gonera, Antje, 1540 Vestby (NO)
(74) Representative: Ricker, Mathias

(57) **Abstract**

The present invention relates to the use of microfibrillated cellulose as an antiwrinkle agent. The present invention also relates to a skin care composition comprising at least one microfibrillated cellulose, said at least one microfibrillated cellulose being characterized by a water holding capacity of at least 75 and to a method for preparing the same.

## Description

The present invention relates to the use of microfibrillated cellulose as an anti-wrinkle agent. The present invention also relates to a skin care composition comprising at least one microfibrillated cellulose and to a method for preparing the same.

### Background of the Invention

Anti-wrinkle and/or anti-aging creams are in increasing demand for everyday use, also under aspects of skin protection. It has therefore become important for cosmetics manufacturers to include anti-wrinkle ingredients in basic cream formulations for creams, ointments and the like.

Skin moisturizers and sunscreens also may be formulated to provide benefits as anti-aging creams.

Examples of anti-wrinkle ingredients that may be used in anti-aging creams are:
- Retinol;
- Epidermal growth factor;
- Alpha hydroxy acids (AHAs) and beta hydroxy acids or other chemical peels;
- Peptides, such as Matryxil and copper peptides;
- Coenzyme Q10;
- Argireline (also known as acetyl hexapeptide-3);
- Anti-oxidants;
- Vitamin C.

The above listed ingredients are of synthetic origin and do not satisfy increasing demands for products of natural origin.

Therefore, it is an object of the present invention to provide a new type of anti-wrinkle agent, which is of natural origin. In particular, a less expensive alternative to the anti-wrinkle agents known from the art is seen as desirable.

Cellulose-based materials are used in anti-aging masks or products, in principle, usually as a swellable media or as the support used to apply the anti-wrinkle ingredient on the desired part of human skin.

US 2004/0156811A1 describes microcrystalline cellulose as an enhancing agent for wrinkle-hiding in decorative skin cosmetics.

Microfibrillar cellulose has been used in personal care products (e.g. body washes, hand soaps and conditioners) mostly as a rheology modifier (as a thickener, a suspending aid and/or as a gel forming agent). WO2013031030A1 describes MFC as a gel forming agent in cosmetic sheets. WO2013094077A1 describes microfibrillar cellulose as building material for cosmetic sheets. EP2307100A2 describes MFC as a rheology modifier in liquid cleanser compositions.

EP1144103A2 describes nanofibrils of amorphous cellulose as an emulsifying agent and/or a dispersion stabilizer. HU9903102A2 describes nanofibrils of amorphous cellulose as a viscosity modifier in cosmetics and detergent products.

### Summary of the Invention

The above mentioned object and other objects are achieved by the teaching of the present invention. Surprisingly, it has been found that microfibrillated cellulose has a pronounced anti-wrinkle effect on human skin.

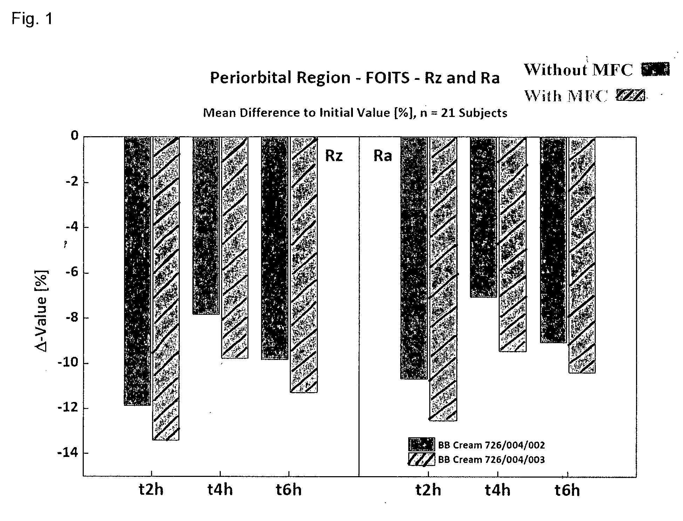

Fast optical in vivo topometry of human skin (FOITS) measurement revealed a pronounced anti-wrinkle effect of microfibrillated cellulose formulated in a beauty balm ('BB') cream on human skin in as short a time span as 2 hours. This effect was also observed at 4 hours and at 6 hours in a kinetic FOITS measurement. The BB cream comprising MFC did show a significantly increased anti-wrinkle effect, at all three time points, compared to the respective BB cream without MFC, as illustrated in Figure 1.

Without wishing to be bound by theory, it is believed that the positive effect observed in this experiment might be due to the fact that MFC is a high molecular weight filling agent. Another characteristic of MFC that might contribute to its anti-wrinkle effect is it comparatively high water holding capacity (water retention capacity). Preferably, the microfibrillated cellulose has a water holding capacity of at least 75, further preferably of at least 80, further preferably of at least 100 as measured in accordance with the method described herein. Without wishing to be bound by theory, it is believed that improved water retention is due to the specific morphology of the MFC fibrils/fibril bundles.

Thus, in a first aspect, the present invention relates to the use of microfibrillated cellulose as an anti-wrinkle agent.

In a second aspect, the present invention relates to a skin care composition comprising at least one microfibrillated cellulose, said at least one microfibrillated cellulose being characterized by a water holding capacity of at least 75.

The present invention further relates to a method of preparing a skin care composition comprising mixing at least one fatty phase and at least one aqueous phase thereby forming an oil in water (O/W) emulsion, said at least one aqueous phase comprising at least one microfibrillated cellulose, said at least one microfibrillated cellulose being characterized by a water holding capacity of at least 75.

Preferred embodiments of the present invention are the subject-matter of the dependent claims.

### Detailed Description of the Invention

In the following, the invention is described with reference to the enclosed figure, wherein:
- **Figure 1**: shows the effect of a BB cream comprising MFC on wrinkles of human skin after 2 hours, 4 hours and 6 hours compared to the effect of a BB cream without MFC.

Advantages, preferred embodiments and possible applications of the present invention are described in the following.

Microfibrillated cellulose (MFC) as used within the claimed composition and in the meaning of the present invention relates to cellulose fibers of any conceivable origin. In particular, MFC according to the present invention relates to cellulose in fiber form that has been subjected to a mechanical treatment in order to increase the fibers' specific surface and to reduce their size in terms of cross-section and of length, wherein said size reduction leads to a fiber diameter in the nanometer range and a fiber length in the micrometer range.

MFC (also known as "microfibrillar cellulose", "reticulated cellulose", "superfine cellulose" or as "cellulose nanofibrils", among others) is prepared from cellulose fibers which are defibrillated using high pressure and/or high mechanical force. In the cellulose starting material (typically present as a "cellulose pulp") no, or at least not a significant or not even a noticeable portion of individualized and "separated" cellulose fibrils can be found. By contrast, in MFC, individual fibrils and fibril bundles can be easily discerned by way of conventional optical microscopy. Sometimes, these fibrils and bundles of fibrils are also described as "(micro)fibrils". In accordance with the present invention, any reference to "fibrils" also includes bundles of such fibrils. Microfibrillated cellulose is described e.g. in US 4 481 077, US 4 374 702 and US 4 341 807. According to US 4 374 702 ("Turbak"), microfibrillated cellulose has properties distinguishable from previously known celluloses.

Due to its large surface area and a high aspect ratio (length to width ratio), microfibrillated cellulose is believed to have a good ability to form rigid networks. In solution, MFC typically forms a highly viscous gel-like dispersion with shear thinning properties. The large surface area of the MFC and the high amount of accessible hydroxyl groups cause MFC to typically have a high water holding capacity.

MFC in accordance with "Turbak" is produced by passing a liquid suspension of cellulose through a small diameter orifice in which the suspension is subjected to a large pressure drop and a high velocity shearing action followed by a high velocity decelerating impact and repeating the passage of said suspension through the orifice until the cellulose suspension becomes a substantially stable suspension. The process converts the cellulose into microfibrillated cellulose without substantial chemical change of the cellulose starting material.

An improved process for obtaining particularly homogeneous MFC is described in WO 2007/091942.

The microfibrillated cellulose in accordance with the present invention may be produced according to any process known in the art. Preferably, said method comprises at least one mechanical step and at least one homogenizing step. The mechanical pretreatment step preferably is or comprises a refining step. The method can also comprise a chemical pretreatment step. One example of such pretreatment step might be oxidation of the hydroxyl groups on the surface of the microfibrils to carboxylic acids. Negative charges of the carboxylic groups cause repulsion between the microfibrils which aids the defibrillation of the cellulose.

The purpose of the mechanical pretreatment step is to "beat" the cellulose pulp in order to increase the accessibility of the cell walls, i.e. increase the surface area and therefore to increase the water retention value. In the refiner that is preferably used in the mechanical pretreatment step, one or two rotating disk(s) is/are employed, i.e. the cellulose pulp slurry is subjected to shear forces.

Prior to the mechanical or chemical pretreatment step, or in between the mechanical or chemical pretreatment steps or as the mechanical pretreatment step, enzymatic (pre)treatment of the cellulose pulp is an optional additional step that may be preferred for some applications. In regard to enzymatic pretreatment in conjunction with microfibrillating cellulose, the respective content of WO 2007/091942 is incorporated herein by reference.

The microfibrillated cellulose preferably has a high aspect ratio which corresponds to the ratio of the length of the microfibril bundle to its diameter (L/D). To this end, the pulp slurry is preferably passed through a homogenizer (a high-pressure homogenizer or a low-pressure homogenizer) and subjected to a pressure drop by forcing the pulp slurry between opposing surfaces, preferably orifices. The term "orifice" means an opening or a nozzle or a valve contained in a homogenizer suitable for homogenizing cellulose.

In particular embodiments, the length and/or the diameter of the cellulose fibrils and fibril bundles of the microfibrillated cellulose are reduced vis-à-vis the respective length and diameter of the cellulose fibers and fiber bundles making up the cellulose that was used as a starting point.

In particular embodiments, the average MFC fibril length is from 100 nm to 50 µm, preferably from 500 nm to 25 µm, more preferably from 1 µm to 10 µm, most preferably from 3 µm to 10 µm.

In particular embodiments, the average MFC fibril diameter is from 1 nm to 500 nm, preferably from 5 nm to 100 nm, more preferably from 10 nm to 50 nm, most preferably from 10 nm to 30 nm.

In particular embodiments, the average MFC fibril aspect ratio is comparatively high.

Fibril length and fibril diameter are determined by imaging using Atomic Force Microscopy and/or Scanning Electron Microscopy.

In particular embodiments, the MFC has a comparatively high specific surface area. Within the context of the present application, the term "specific surface area" refers to the total surface area of an MFC material per unit of mass.

In particular embodiments, the microfibrillated cellulose has a water holding capacity (water retention capacity) of at least 75, preferably at least 80, further preferably at least 100. The water holding capacity describes the ability of the MFC to retain water within the MFC structure and this again relates to the accessible surface area. According to the invention, the water holding capacity is measured by diluting an MFC sample to a 0.3% solids content in water and then centrifuging the sample at 1000 G for 15 minutes. The clear water phase is separated from the sediment and the sediment is weighed. The water holding capacity is given as (mV/mT)-1 where mV is the weight of the wet sediments and mT is the weight of dry MFC analyzed.

Without wishing to be bound by theory, it is believed that the bundles of fibrils might have a matting effect on skin hiding its flaws. This may allow the skin care compositions to give the skin a matte finish and to conceal small flaws or blemishes thereby resulting in an anti-wrinkle effect.

In principle, the raw material for the cellulose microfibrils may be any cellulosic material, in particular cellulose originating from wood, annual plants, cotton, flax, straw, ramie, sugar cane bagasse, certain algae, jute, sugar beet, citrus fruits, waste from the food processing industry or energy crops or cellulose of bacterial or animal origin, e.g. from tunicates.

In particular embodiments, wood-based materials are used as raw materials, either hardwood or softwood or both (in mixtures). Further preferably softwood is used as a raw material, either one kind or mixtures of different soft wood types.

The term MFC, in accordance with the present invention, encompasses a single kind of microfibrillated cellulose as well as a mixture of structurally different microfibrillated celluloses, such as a mixture of unmodified and chemically and/or physically modified microfibrillated cellulose.

The microfibrillated cellulose in accordance with the present invention may be unmodified with respect to its functional group or may be physically modified or chemically modified, preferably resulting in neutral or negatively charged groups on the microfibril's surface, or both. In one embodiment, the MFC, in accordance with the present invention, is substantially free of cationic substituents.

The preferred embodiment of chemical modification of the surface of the cellulose microfibrils in the present invention is preferably achieved by various reactions of surface functional groups of the cellulose microfibrils and more particularly of the hydroxyl functional groups, preferably resulting in neutral or negatively charged groups on the microfibril surface. Examples of such reactions are oxidation, silylation reactions, etherification reactions, condensations with isocyanates, alkoxylation reactions with alkylene oxides or condensation or substitution reactions with glycidyl derivatives. Chemical modification may take place before or after the defibrillation step. Thus, in particular embodiments, the MFC is chemically modified by oxidation, silylation, etherification, condensation with isocyanates, alkoxylation with alkylene oxides, condensation with glycidyl derivatives, substitution with glycidyl derivatives or any combination thereof.

The cellulose microfibrils may also be modified by a substantially 'physical' method, which does not involve the formation of chemical bonds to a substantial extend, in particular, either by adsorption at the surface, or by spraying, coating or encapsulation of the microfibril. Preferred modified microfibrils can be obtained by physical adsorption of at least one compound. The microfibrils can be modified by physical adsorption of at least one amphiphilic compound (surfactant).

In one embodiment, microfibrils are modified by physical adsorption of at least one non-ionic surfactant. EP2 408 857 describes processes of preparing surface modified MFC. The physical modification of the MFC surface may take place before or after the defibrillation step.

Physical modification of the cellulose microfibril surface may occur prior to the mechanical pretreatment step, between the mechanical pretreatment step and the defibrillation step or after the defibrillation step.

The microfibrillated cellulose (MFC) according to the present invention may be subjected to at least one dewatering and/or drying step. The at least one drying step is preferably selected from freeze-, spray- or roller-drying, drying in a convection oven, flash drying or the like. 'Never-dried' microfibrillated cellulose may also be used and the microfibrillated cellulose used in the present invention might have a dry content ranging from 0.1%-100% before it is added to a skin care composition.

In particular embodiments, the dry content of the MFC is from 0.1% to 100%, preferably from 0.2% to 40%, more preferably from 0.5% to 25%, even more preferably from 1% to 20%, before the MFC is added to the composition.

Within the context of the present application, the term "anti-wrinkle agent" refers to a substance having wrinkle reducing effects on human skin, meaning that the depth of at least a portion of the wrinkles that are contacted with the anti-wrinkle agent is reduced to some extend for a certain period of time. For example, the average wrinkle depth may be reduced by 0.2%, 0.5%, 1%, 2%, 5%, 10%, 15; 20% or more instantly or after 1 h, 2 h, 3 h, 6 h, 12 h or 24 h after application of the anti-wrinkle agent. The wrinkle reducing effect may persist for 2 h, 4 h, 6 h, 8 h, 10 h, 12 h, 24 h, 36 h or longer. For example, a 'wrinkle reducing' effect exists, if a reduction of the average wrinkle depth by at least 0.2% is observed 2 h after the application of the anti-wrinkle agent and persists for at least a further 2 h.

According to one embodiment, the microfibrillated cellulose is provided in a skin care composition. These skin care compositions are preferably used for topical application onto the skin. The cosmetic composition is preferably semi-solid at room temperature.

The MFC comprising composition may be in any suitable form commonly used in cosmetics. In particular embodiments, the composition is selected from a cream, a lotion, a paste, an emulsion, a gel, a foundation, a serum, and an ointment. Particularly preferred is the addition of microfibrillated cellulose to beauty balm creams. In accordance with the present invention, the term "beauty balm cream" or "BB cream" refers to an all-in-one facial cosmetic product which may replace multiple simultaneously used cosmetic products. BB creams have moisturizing effects, sun protection effects and some also 'anti-aging' properties. They are generally suitable to replace skin moisturizers, foundation as well as sunscreens.

Accordingly, in particular embodiments, the skin care composition further comprises at least one skin moisturizer and at least one UV filter.

Within the context of the present application, the term "moisturizer" refers to a dermatologically compatible agent that helps to restore moisture or to retain moisture in the skin upon application. Suitable moisturizers may be selected from, but are not limited to: naturally occurring skin/membrane lipids and sterols (steroid alcohols), artificial or natural oils, humectants, emollients, and lubricants.

Within the context of the present application, the term "UV filter" refers to an agent able to block, absorb and/or reflect UV rays. Suitable UV filters may be selected from, but are not limited to, Zinc Oxide, Titanium Dioxide, Avobenzone, Tinosorb S, Tinosorb M, Mexoryl SX, Mexoryl XL, Helioplex, Octinoxate, Octocrylene, Oxybenzone, Octisalate, Homosalate, Uvinul T 150, Cinoxate, Aminobenzoic acid, Padimate O, Ensulizole, Dioxybenzone, Meradimate, Sulisobenzone, Trolamine salicylate, Enzacamene, Bisdisulizole Disodium, Uvinul A Plus, Uvasorb HEB, Parsol SLX and Amiloxate.

The skin care composition may include further components or ingredients which are compatible with human skin. The ingredients may be chosen according to the selected application of the product and may be chosen from, but are not limited to, excipients, surfactants, emulsifiers, thickeners, vitamins, fats, oils, waxes, humectants, UV filters, preservatives, water, alcohol, perfumes, anti-foaming agents, foam stabilizers, polymers, electrolytes, organic solvents, silicone derivatives and coloring agents.

The formulation of pharmaceutically-acceptable dermatological and cosmetic preparations, in principle, is known in the art.

In particular embodiments, the concentration of the microfibrillated cellulose in the skin care composition is from 0.04% to 25% by weight, preferably from 0.1 to 10% by weight, more preferably from 0.2 to 5% by weight, most preferably from 0.5 to 2% by weight.

In a second aspect, the present invention relates to a skin care composition comprising at least one microfibrillated cellulose, said at least one microfibrillated cellulose being characterized by a water holding capacity of at least 75.

In particular embodiments, the skin care composition is an oil in water (O/W) emulsion, particularly a beauty balm cream. In particular embodiments, the skin care composition further comprises titanium dioxide as UV filter.

In a third aspect, the present invention relates to a method of preparing a skin care composition comprising mixing at least one fatty phase and at least one aqueous phase thereby forming an oil in water (O/W) emulsion, said at least one aqueous phase comprising at least one microfibrillated cellulose, said at least one microfibrillated cellulose being characterized by a water holding capacity of at least 75.

In particular embodiments, the method of preparing a skin care composition is a method of preparing a beauty balm cream. In particular embodiments, the beauty balm cream further comprises titanium dioxide. In particular embodiments, the method of preparing a beauty balm cream comprises preparing an oil in water (O/W) emulsion by mixing a fatty phase (phase A in table 1), titanium dioxide for sun protection (phase B in table 1) and an aqueous phase comprising at least one microfibrillated cellulose (phase C in table 1).

### Examples

### FOITS test measurements carried out on BB creams with and without MFC

MFC was tested for its anti-wrinkle properties in a kinetic measurement test using FOITS as the measurement method. In order to perform the test, 1% (dry material) MFC was formulated in a Beauty Balm BB cream. A paired comparison against Placebo formulation without MFC on 20 female volunteers was carried out. Formulation work and FOITS measurements were carried out in the laboratories of Institute Dr. Schrader, Max-Planck-Straße 6, D-37603 Holzminden - Germany.

### Preparation of the BB creams

The BB creams used in these tests, BB cream 726/004/002 and BB cream 726/004/003, contain the ingredients listed in Table 1.

The Beauty balm creams were prepared on a laboratory scale of approximately 500 g using standard stirrers and dissolvers. Titanium dioxide was dispersed in the hot fatty phase by a high shear Ultra Turrax™. The MFC was dispersed at room temperature in the aqueous phase using a dissolver. The aqueous phase was then heated to 80°C. After emulsification, the emulsion was finally homogenized in a Homozenta™ emulsifying machine.

**Table 1.**

| Pos. | Raw Material | INCI (Eu) | Supplier | % [w/w] 726/004/002 | % [w/w] 726/004/003 |
|---|---|---|---|---|---|
| Phase A | | | | | |
| 1 | Amphisol K | Potassium Cetyl Phosphate | Nordmann Rassmann | 2.20 | 2.20 |
| 2 | Lanette O | Cetearyl Alcohol | BTC Europe GmbH | 2.00 | 2.00 |
| 3 | Tegin M Pellets | Glyceril Stearate | Goldschmid t Evonik | 1.00 | 1.00 |
| 4 | Softisan 100 | Hydrogenated Coco glycerides | Sasol Germany GmbH | 2.00 | 2.00 |
| 5 | Eutanol G | Octyldodecanol | BTC Europe GmbH | 5.00 | 5.00 |
| 6 | Isopropylpalmitat (IPP) | Isopropyl Palmitate | BTC Europe GmbH | 8.00 | 8.00 |
| 7 | Neo Heliopan 303 (600154) | Octorylene | Symrise | 2.00 | 2.00 |
| 8 | all-rac-alpha-Tocopherylacetat (Art. 500952) | Tocopheryl Acetate | Merck | 0.50 | 0.50 |
| 9 | Euxyl PE 9010 | Phenoxyethanol, Ethylhexylglycerin, Tocopherol | Schülke & Mayr | 1.00 | 1.00 |

| Phase B | | | | | |
|---|---|---|---|---|---|
| 10 | Eusolex T-AVO (105335) | Titanium Dioxide (Nano), Silica | Merck KGaA | 3.00 | 3.00 |
| 11 | Kronos 1171 (Titandioxid, 260 nm) | CI 77891 | Kronos Titan | 3.00 | 3.0 |
| 12 | Symcolor Eisenoxidgelb 656835 | CI 77492 | Symrise | 0.70 | 0.70 |
| 13 | Dragocolor Eisenoxidrot E172 656836 | CI 77491 | Symrise | 0.16 | 0.16 |
| 14 | Dragocolor Eisenoxidschwarz 656837 | CI 77499 | Symrise | 0.08 | 0.08 |

| Phase C | | | | | |
|---|---|---|---|---|---|
| | | | | | |
| 15 | Water, demin. | AQUA | | 64.91 | 54.91 |
| 16 | Edeta BD | Disodium EDTA | BTC Europe GmbH | 0.05 | 0.05 |
| 17 | Glycerin Ph. Eur. 85% | Glycerin, Aqua | Azelis | 4.00 | 4.00 |
| 18 | Keltrol CG-SFT | Xanthan Gum | Rahn AG | 0.40 | 0.40 |
| 19 | MFC-Paste m/Phenoxyethanol 10% active matter | | Borregaard | -- | 10.00 |
| | | | | 100.00 | 100.00 |

Microfibrillated cellulose can be prepared as described herein.

The cream was applied to the skin and the FOITS measurements of the Periorbital region were recorded.

### Fast Optical in Vivo Topometry of human skin (FOITS)

The three-dimensional skin structure was determined by FOITS measurements. The FOITS method is a non-contact method of three-dimensional wrinkle analysis based on an optical measuring technique (fringe projection) for three-dimensional data collection providing an objective touch free, non-invasive, in vivo assessment of skin microtopography. The FOITS methodology allows for full 3D-visualisation of the anti-wrinkle or anti-aging effect and statistical analysis of roughness parameters Rz and Ra according to the expanded version of DIN 4768.

After measuring the initial situation (t0), the measurements were repeated after two (t2h), four (t4h) and six hours (t6h).

### Periorbital Region - FOITS - Rz and Ra - Summary and Statistics

Rz represents a measure of the peak to valley size of the largest features (largest wrinkles). Ra is a measure of the overall residual difference from profile centerline (overall roughness).

Table 2 and Figure 1 show the mean values obtained for Rz and Ra at initial time of application of both BB creams, after 2h, after 4h and after 6h. The statistical analysis of the data shows a significant difference between the values obtained by the Bb cream without MFC and that with MFC.

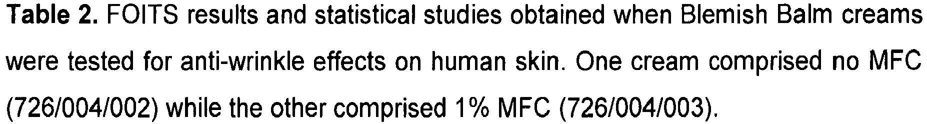

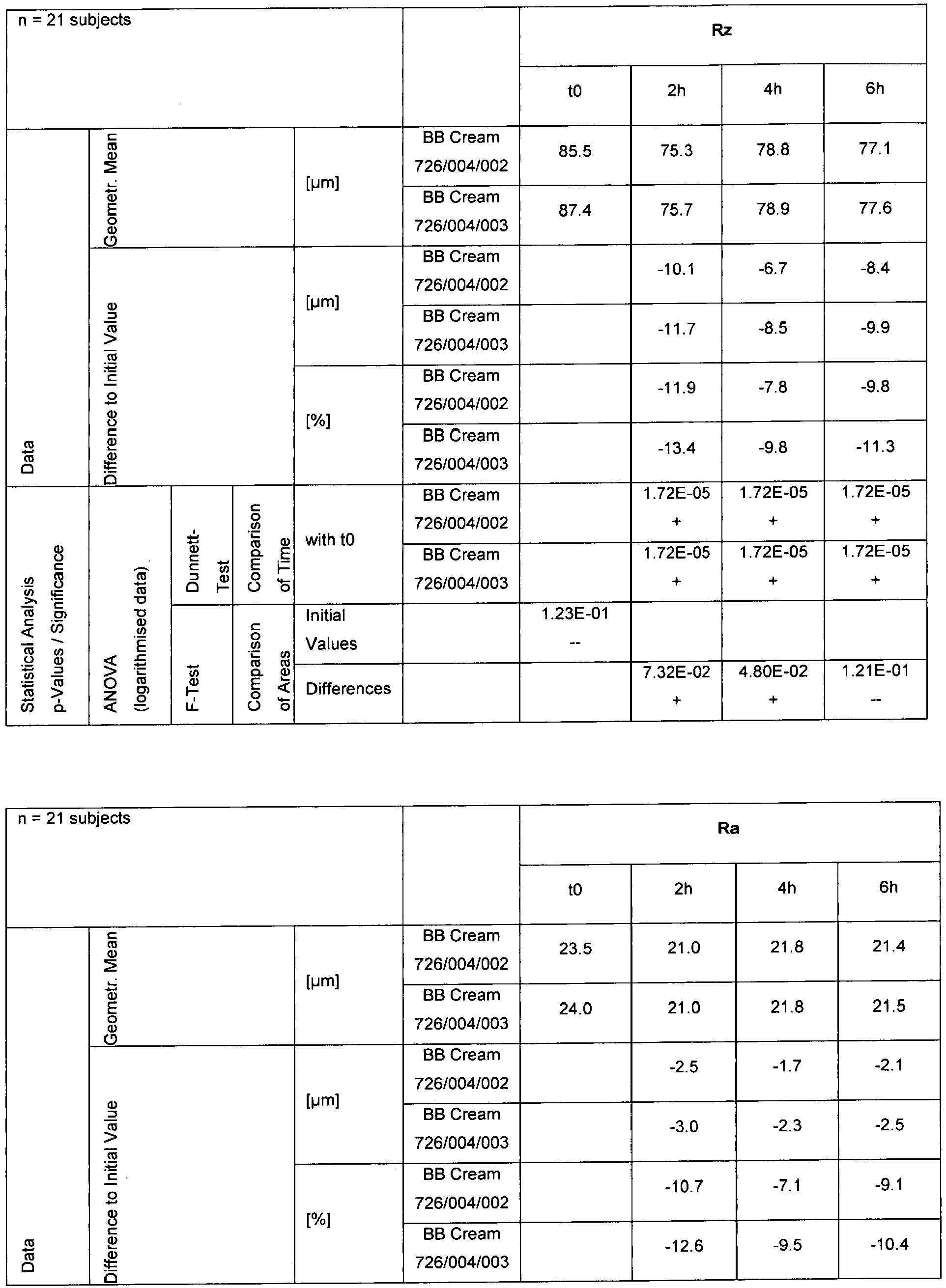

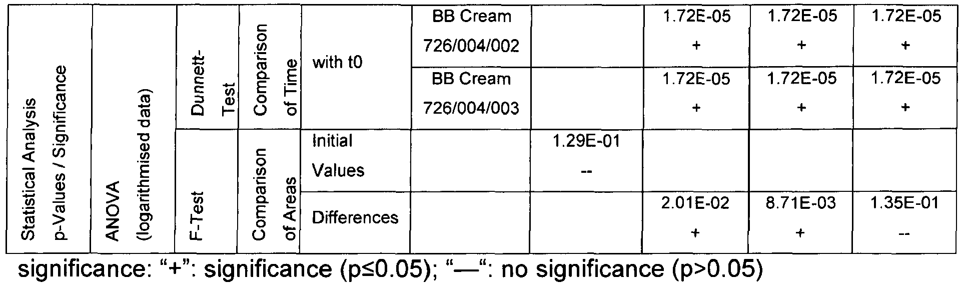

BB cream 726/004/003 (with MFC) and the control 726/004/002 (without MFC) both showed a significant anti-wrinkle effect after 2 h, 4 h and 6 h compared to time t0 because both BB creams comprise several other care components (lipids) and moisturizers (water, glycerol etc.).

The BB cream with MFC showed a significantly increased anti-wrinkle effect after 2 h, 4 h as well as 6 h compared to the BB cream without MFC. Figure 1 clearly illustrates the pronounced anti-wrinkle effect of the cream with MFC compared to the cream without MFC.

## Claims

1. Use of microfibrillated cellulose (MFC) as an anti-wrinkle-agent.

2. The use according to claim 1, wherein the MFC is provided in a skin care composition, preferably selected from a cream, a lotion, a paste, an emulsion, a gel, a foundation, a serum, and an ointment.

3. The use according to claim 1 or 2, wherein the MFC is an unmodified MFC or a chemically modified MFC, preferably having neutral or negatively charged substituents, or a physically modified MFC or any combination thereof.

4. The use according to any one of the preceding claims, wherein the average MFC fibril length is from 100 nm to 50 µm, preferably from 500 nm to 25 µm, more preferably from 1 µm to 10 µm, most preferably from 3 µm to 10 µm.

5. The use according to any one of the preceding claims, wherein the average MFC fibril diameter is from 1 nm to 500 nm, preferably from 5 nm to 100 nm, more preferably from 10 nm to 50 nm, most preferably from 10 nm to 30 nm.

6. The use according to any one of the preceding claims, where the water holding capacity of the MFC is at least 75, preferably at least 80, more preferably at least 100.

7. The use according to any one of the preceding claims, wherein the MFC is unmodified MFC.

8. The use according to any one of the preceding claims, wherein the MFC is chemically modified by oxidation, silylation, etherification, condensation with isocyanates, alkoxylation with alkylene oxides, condensation with glycidyl derivatives, substitution with glycidyl derivatives or any combination thereof.

9. The use according to any one of the preceding claims, wherein the MFC is physically modified by physical absorption of at least one amphiphilic compound, preferably by a non-ionic amphiphilic compound.

10. The use according to any one of the preceding claims, wherein the MFC is substantially free of cationic substituents.

11. The use according to any one of claims 2 to 10, wherein the dry content of the MFC is from 0.1 % to 100%, preferably from 0.2% to 40%, more preferably from 0.5% to 25%, more preferably from 1% to 20%, before the MFC is added to the composition.

12. The use according to any one of claims 2 to 11, wherein the concentration of the MFC in the skin care composition is from 0.04% to 25% by weight, preferably from 0.1 to 10% by weight, more preferably from 0.2 to 5% by weight, most preferably from 0.5 to 2% by weight.

13. The use according to any one of claims 2 to 12, wherein the composition further comprises at least one skin moisturizer and at least one UV filter.

14. Skin care composition comprising at least one microfibrillated cellulose, said at least one microfibrillated cellulose being **characterized by** a water holding capacity of at least 75.

15. A method of preparing a skin care composition comprising mixing at least one fatty phase and at least one aqueous phase thereby forming an oil in water (O/W) emulsion, said at least one aqueous phase comprising at least one microfibrillated cellulose, said at least one microfibrillated cellulose being **characterized by** a water holding capacity of at least 75.
